# EUROPEAN PATENT APPLICATION

(11) **EP 3 932 918 A1**
(43) Date of publication of application: **05.01.2022**
(21) Application number: 19946891.9
(22) Date of filing: 13.12.2019
(51) Int. Cl.: C07D 471/04, C07C 51/43, C07C 55/08, C07C 57/02, C07C 59/255, C07D 207/16

(54) **EUTECTIC CRYSTAL FORMED BY APIXABAN AND CARBOXYLIC ACID, AND PREPARATION METHOD THEREFOR**

(30) Priority: 26.09.2019 CN 201910919747
(71) Applicant: Zhejiang Tianyu Pharmaceutical Co., Ltd., Huangyan Taizhou Zhejiang 318020 (CN)
(72) Inventor: ZHU, Guorong, Zhejiang 318020 (CN); KE, Chunlong, Zhejiang 318020 (CN); WANG, Zhen, Zhejiang 318020 (CN); TU, Yongjun, Zhejiang 318020 (CN); ZHANG, Peng, Zhejiang 318020 (CN)
(74) Representative: Dehns
(86) International application number: PCT/CN2019/125115
(87) International publication number: WO 2021/056850

(57) **Abstract**

Provided are a co-crystal formed by Apixaban and a carboxylic acid, and a preparation method therefor. The co-crystal comprises Apixaban/malonic acid co-crystal, Apixaban/D-malic acid co-crystal, Apixaban/maleic acid co-crystal, Apixaban/L-proline co-crystal, and Apixaban/L-tartaric acid co-crystal. The co-crystal solves the disadvantages of slow dissolving speed and low dissolution rate of Apixaban, has an improved dissolution rate of Apixaban, and is beneficial to improve bioavailability.

## Description

### TECHNICAL FIELD

The present invention relates to the field of medicinal chemistry, in particular to Apixaban/carboxylic acid co-crystal and a preparation method therefor.

### BACKGROUND

Apixaban, which has an English name of Apixaban (trade name: Eliquis), a chemical name of 4,5,6,7-tetrahydro-1-(4-methoxyphenyl)-7-oxo-6-[4-(2-oxo-1-piperidinyl)phenyl]-1H-pyrazolo [3,4-c]pyridine-3-carboxamide, CAS: 503612-47-3, and a chemical structure as follows:

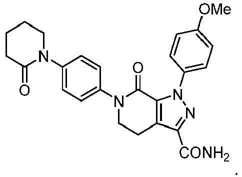

Apixaban is a new generation of antithrombotic drugs. It is a new direct inhibitor of factor Xa jointly developed by Bristol-Myers Squibb and Pfizer. The product is currently clinically used to prevent Venous thromboembolism (VTE) in adult patients who have undergone elective hip or knee replacement surgery.

Apixaban is almost insoluble in water, and has the disadvantages of slow dissolving speed and unfavorable drug absorption in the body. There is an urgent need to find a way to improve the dissolution rate of Apixaban. In order to solve this problem, patents CN101065379, US20070203178 and CN104086544 provide a variety of crystal forms of Apixaban, including hydrates and solvates. However, these crystal forms are not effective in improving the dissolution rate of Apixaban.

Patent CN106986868 discloses co-crystals formed by Apixaban with oxalic acid, isonicotine, 3-aminopyridine and urea. These co-crystals have improved the dissolution rate of apixaban to a certain extent. However, the small molecules of bases such as isonicotine, 3-aminopyridine and urea in the co-crystals disclosed in the patent have certain side effects on the human body. For example, 3-aminopyridine is a potential genotoxic impurity. These unfavorable factors are not conducive to the development of the aforementioned co-crystals as oral preparations that are safe for humans.

### SUMMARY

The purpose of the present invention is to overcome the shortcomings of the prior art and develop new types of Apixaban/carboxylic acid co-crystal with a specific molecular ratio and a preparation method therefor. The co-crystal can improve the dissolution rate of Apixaban while meeting the requirement of safe medication, solve the disadvantages of slow dissolving speed and low dissolution rate of Apixaban, and improve its bioavailability.

The present invention provides a co-crystal formed by Apixaban and a carboxylic acid, wherein the molar ratio of the Apixaban to the carboxylic acid is 1:0.5.

In the co-crystal formed by Apixaban and carboxylic acid of the present invention, the carboxylic acid is malonic acid, D-malic acid, maleic acid, L-proline or L-tartaric acid, that is, these five co-crystals are Apixaban/malonic acid co-crystal, Apixaban/D-malic acid co-crystal, Apixaban/maleic acid co-crystal, Apixaban/L-proline co-crystal and Apixaban/L-tartaric acid co-crystal, correspondingly called crystal form I, crystal form II, crystal form III, crystal form IV and crystal form V.

In the co-crystal formed by Apixaban and a carboxylic acid of the present invention, the structural formula of Apixaban/malonic acid co-crystal form I is shown in the following Formula 1:

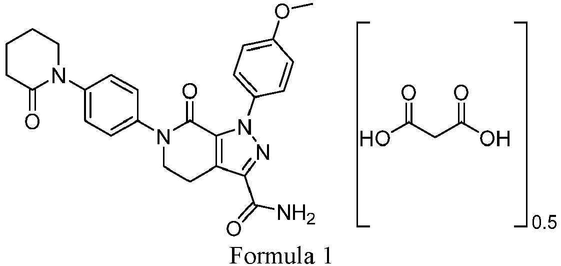

The crystal form I has the following characteristics:
By using Cu-Ka radiation, the X-ray powder diffraction of the crystal form I has characteristic peaks at the positions where the diffraction angles 2θ are 5.5°±0.2°, 5.7°±0.2°, 5.9°±0.2°, 11.0°±0.2°, 11.5°±0.2°, 11.9°±0.2° and 21.0°±0.2°.

Further, the X-ray powder diffraction of the crystal form I has characteristic peaks at the positions where the diffraction angles 2θ are 5.5°±0.2°, 5.7°±0.2°, 5.9°±0.2°, 7.1°±0.2°, 11.0°±0.2°, 11.5°±0.2°, 11.9°±0.2°, 15.5°±0.2°, 15.7°±0.2°, 16.0°±0.2°, 17.4°±0.2°, 21.0°±0.2° and 21.4°±0.2°.

Furthermore, the X-ray powder diffraction of the crystal form I has characteristic peaks at the positions where the diffraction angles 2θ are 5.5°±0.2°, 5.7°±0.2°, 5.9°±0.2°, 7.1°±0.2°, 11.0°±0.2°, 11.5°±0.2°, 11.9°±0.2°, 13.3°±0.2°, 13.5°±0.2°, 15.5°±0.2°, 15.7°±0.2°, 16.0°±0.2°, 17.4°±0.2°, 19.1°±0.2°, 20.2°±0.2°, 21.0°±0.2°, 21.4°±0.2°, 21.8°±0.2°, 22.4°±0.2° and 24.1°±0.2°.

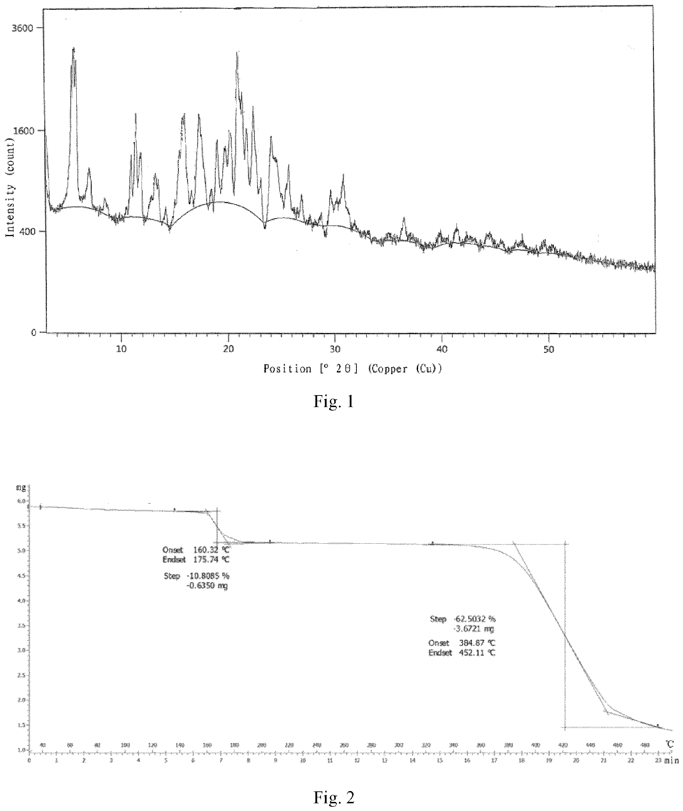

Without limitation, the X-ray powder diffraction pattern of the crystal form I is shown in Fig. 1.

Without limitation, the crystal form I loses 10.80% of its weight at 160°C (losing malonic acid molecules), according to the theoretical calculation, the molar ratio of Apixaban to malonic acid is 1:0.5, and its thermogravimetric analysis (TGA) graph is shown in Fig. 2.

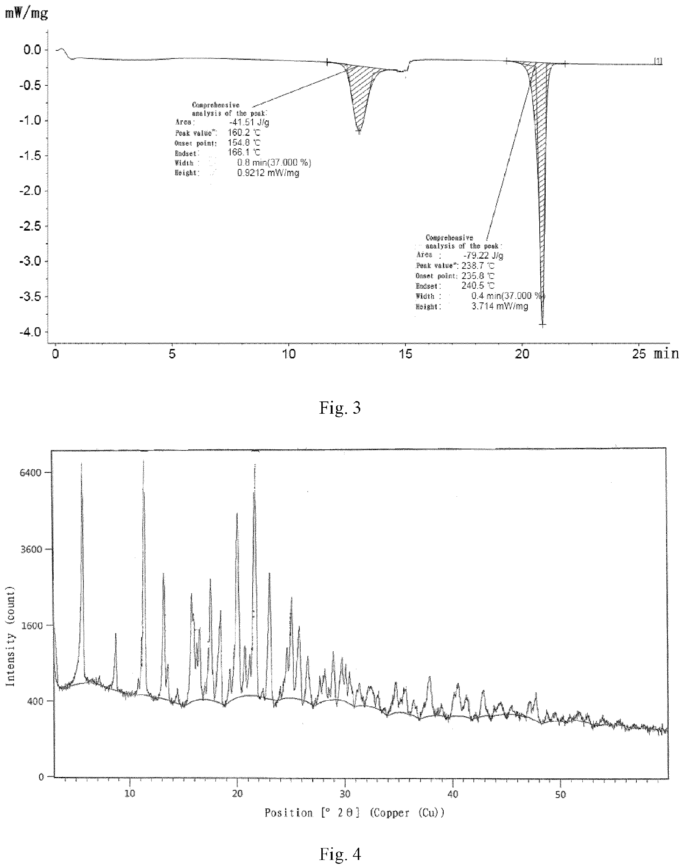

Without limitation, the crystal form I shows two endothermic peaks at about 160.2°C and 238.7°C, and its differential scanning calorimetry (DSC) graph is shown in Fig. 3.

The crystal form I is a powder with good appearance and fluidity, its dissolution rate is 1.4 times that of Apixaban, and is suitable for being prepared into its oral preparation.

In the co-crystal formed by Apixaban and a carboxylic acid of the present invention, the structural formula of Apixaban/D-malic acid co-crystal form II is shown in the following Formula 2:

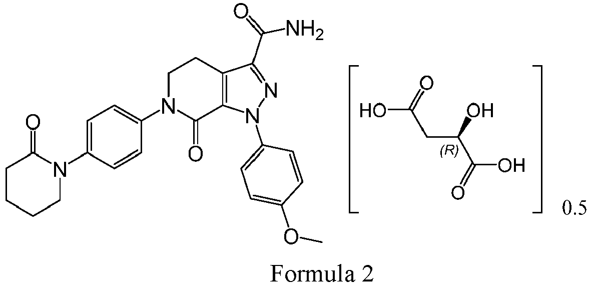

The crystal form II has the following characteristics:
By using Cu-Ka radiation, the X-ray powder diffraction of the crystal form II has characteristic peaks at the positions where the diffraction angles 2θ are 5.7°±0.2°, 8.7°±0.2°, 11.5°±0.2°, 13.2°±0.2°, 17.6°±0.2°, 20.1°±0.2° and 21.8°±0.2°.

Further, the X-ray powder diffraction of the crystal form II has characteristic peaks at the positions where the diffraction angles 2θ are 5.7°±0.2°, 8.7°±0.2°, 11.5°±0.2°, 13.2°± 0.2°, 15.8°±0.2°, 16.0°±0.2°, 17.6°±0.2°, 18.5°±0.2°, 20.1°±0.2°, 21.8°±0.2°, 23.1°±0.2° and 25.1°±0.2°.

Furthermore, the X-ray powder diffraction of the crystal form II has characteristic peaks at the positions where the diffraction angles 2θ are 5.7°±0.2°, 8.7°±0.2°, 11.5°±0.2°, 13.2°± 0.2°, 15.8°±0.2°, 16.0°±0.2°, 16.2°±0.2°, 16.5°±0.2°, 17.6°±0.2°, 18.5°±0.2°, 20.1°±0.2°, 21.8°±0.2°, 23.1°±0.2°, 25.1°±0.2° and 25.8°±0.2°.

Without limitation, the X-ray powder diffraction pattern of the crystal form II is shown in Fig. 4.

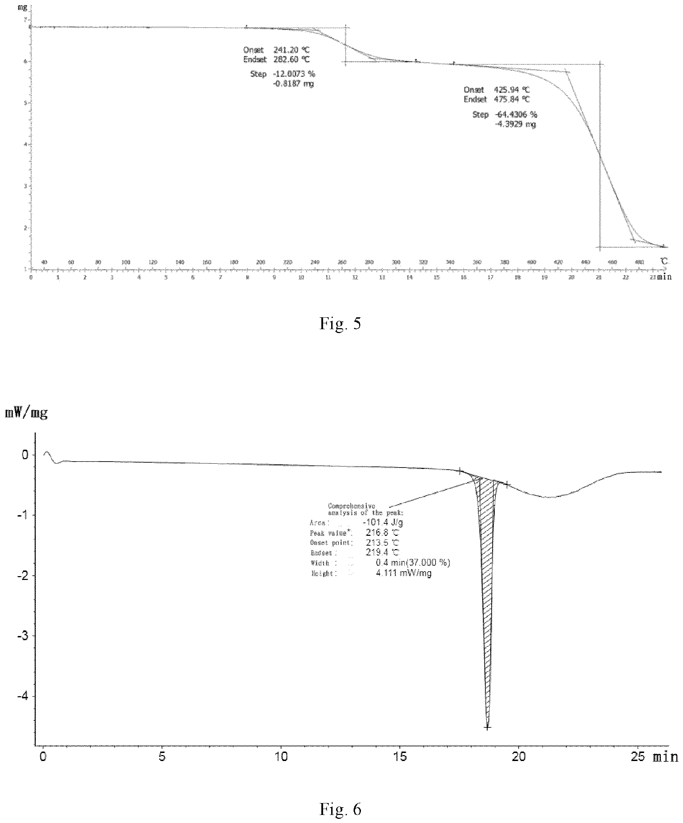

Without limitation, the crystal form II loses 12.01% of its weight at 241°C (losing D-malic acid molecules), according to the theoretical calculation, the molar ratio of Apixaban to D-malic acid is 1:0.5, and its TGA graph is shown in the Fig. 5.

Without limitation, the crystal form II has an endothermic peak at about 216.8 °C, and its DSC graph is shown in Fig. 6.

The crystal form II is a powder with good appearance and fluidity, its dissolution rate is 1.44 times that of Apixaban, and is suitable for being prepared into its oral preparation.

In the co-crystal formed by Apixaban and a carboxylic acid of the present invention, the structural formula of Apixaban/maleic acid co-crystal form III is shown in the following Formula 3:

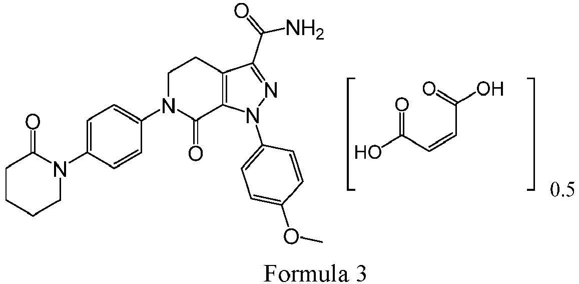

The crystal form III has the following characteristics:
By using Cu-Ka radiation, the X-ray powder diffraction of the crystal form III has characteristic peaks at the positions where the diffraction angles 2θ are 5.5°±0.2°, 5.7°±0.2°, 6.0°±0.2°, 7.2°±0.2°, 11.5°±0.2°, 15.8°±0.2°, 16.1°±0.2°, 17.4°±0.2° and 21.0°±0.2°.

Further, the X-ray powder diffraction of the crystal form III has characteristic peaks at the positions where the diffraction angles 2θ are 5.5°±0.2°, 5.7°±0.2°, 6.0°±0.2°, 7.2°±0.2°, 11.5°±0.2°, 11.9°±0.2°, 13.3°±0.2°, 13.5°±0.2°, 15.8°±0.2°, 16.1°±0.2°, 17.4°±0.2°, 19.1°±0.2°, 19.9°±0.2°, 20.3°±0.2° and 21.0°±0.2°.

Furthermore, the X-ray powder diffraction of the crystal form III has characteristic peaks at the positions where the diffraction angles 2θ are 5.5°±0.2°, 5.7°±0.2°, 6.0°±0.2°, 7.2°± 0.2°, 11.5°±0.2°, 11.9°±0.2°, 13.3°±0.2°, 13.5°±0.2°, 15.8°±0.2°, 16.1°±0.2°, 17.4°±0.2°, 19.1°±0.2°, 19.9°±0.2°, 20.3°±0.2°, 21.0°±0.2°, 21.9°±0.2°, 22.5°±0.2° and 24.1°±0.2°.

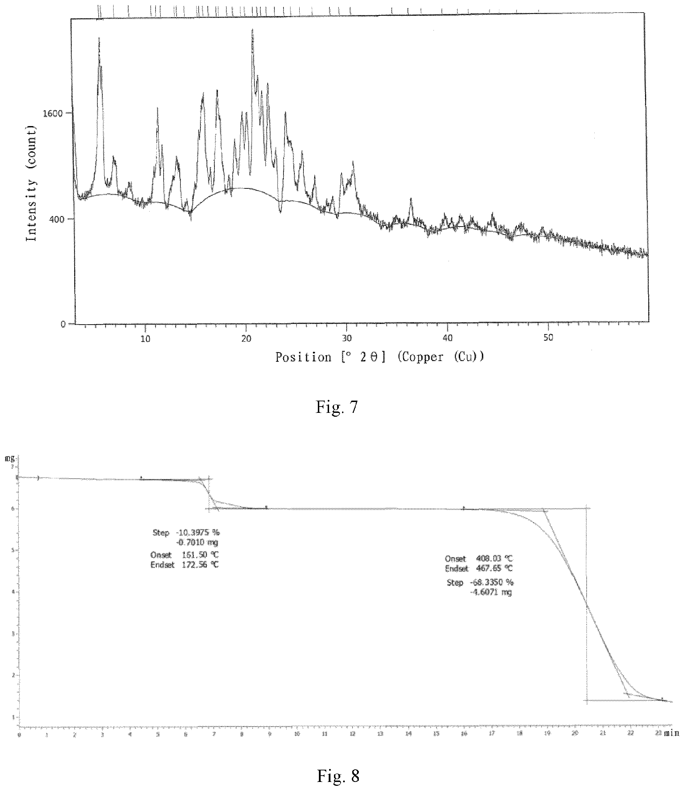

Without limitation, the X-ray powder diffraction pattern of the crystal form III is shown in Fig. 7.

Without limitation, the crystal form III loses 10.40% of its weight at 161°C (loss of maleic acid molecules), according to the theoretical calculation, the molar ratio of Apixaban to maleic acid is 1:0.5, and its TGA graph is shown in Fig. 8.

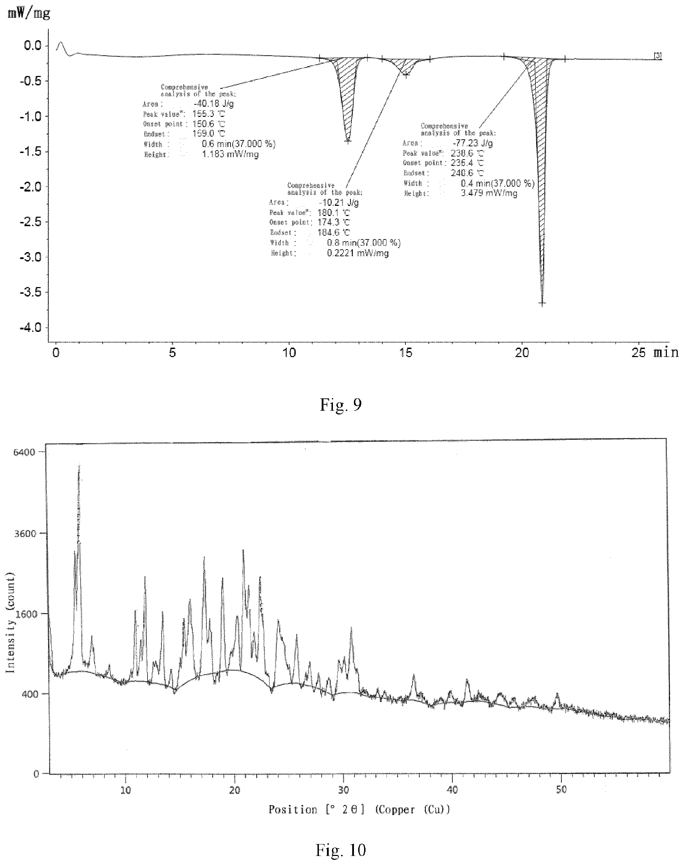

Without limitation, the crystal form III shows endothermic peaks at about 155.3°C, 180.1°C, and 238.6°C, and its DSC graph is shown in Fig. 9.

The crystal form III is a powder with good appearance and fluidity, its dissolution rate is 1.53 times that of Apixaban, and is suitable for being prepared into its oral preparation.

In the co-crystal formed by Apixaban and a carboxylic acid of the present invention, the structural formula of Apixaban/L-proline co-crystal form IV is shown in the following Formula 4:

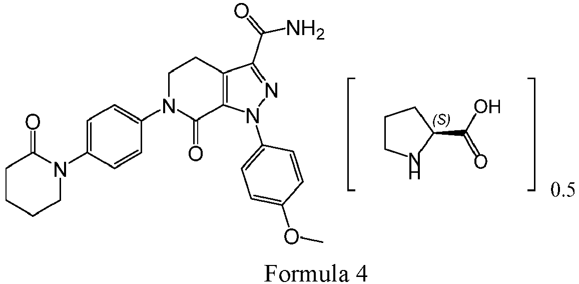

The crystal form IV has the following characteristics:
By using Cu-Ka radiation, the X-ray powder diffraction of the crystal form IV has characteristic peaks at the positions where the diffraction angles 2θ are 5.5°±0.2°, 5.7°±0.2°, 5.9°±0.2°, 7.0°±0.2°, 11.9°±0.2°, 17.4°±0.2°, 19.0°±0.2° and 21.0°±0.2°.

Further, the X-ray powder diffraction of the crystal form IV has characteristic peaks at the positions where the diffraction angles 2θ are 5.5°±0.2°, 5.7°±0.2°, 5.9°±0.2°, 7.0°±0.2°, 11.0°±0.2°, 11.9°±0.2°, 13.5°±0.2°, 15.5°±0.2°, 16.1°±0.2°, 17.4°±0.2°, 17.8°±0.2°, 19.0°±0.2° and 21.0°±0.2°.

Furthermore, the X-ray powder diffraction of the crystal form IV has characteristic peaks at the positions where the diffraction angles 2θ are 5.5°±0.2°, 5.7°±0.2°, 5.9°±0.2°, 7.0°±0.2°, 11.0°±0.2°, 11.9°±0.2°, 13.5°±0.2°, 15.5°±0.2°, 16.1°±0.2°, 17.4°±0.2°, 17.8°±0.2°, 19.0°±0.2°, 21.0°±0.2°, 21.5°±0.2°, 22.5°±0.2° and 24.1°±0.2°.

Without limitation, the X-ray powder diffraction pattern of the crystal form IV is shown in FIG. 10.

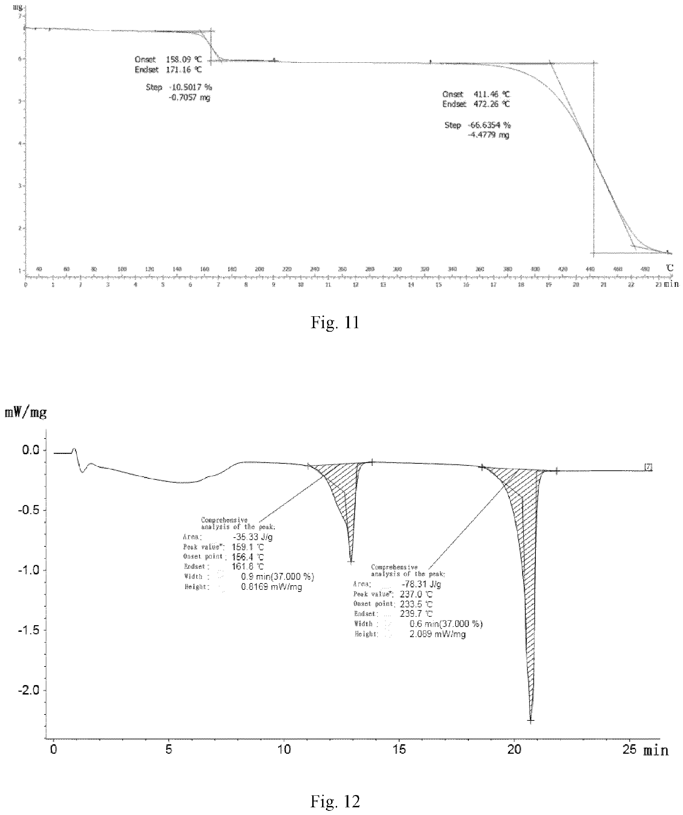

Without limitation, the crystal form IV loses 10.50% of its weight at 158°C (losing L-proline molecules), according to the theoretical calculation, the molar ratio of Apixaban to L-proline is 1:0.5, and its TGA graph is shown in Fig. 11.

Without limitation, the crystal form IV shows endothermic peaks at about 159.1°C and 237.0°C, and its DSC graph is shown in Fig. 12.

The crystal form IV is a powder with good appearance and fluidity, its dissolution rate is 1.54 times that of Apixaban, and is suitable for being prepared into its oral preparation.

In the co-crystal formed by Apixaban and a carboxylic acid of the present invention, the structural formula of Apixaban/L-tartaric acid co-crystal form V is shown in Formula 5 below:

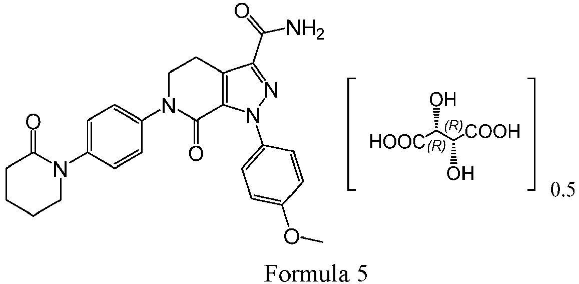

The crystal form V has the following characteristics:
By using Cu-Ka radiation, the X-ray powder diffraction of the crystal form V has characteristic peaks at the positions where the diffraction angles 2θ are 5.5°±0.2°, 7.1°±0.2°, 8.6°±0.2°, 11.0°±0.2°, 17.9°±0.2°, 19.1°±0.2° and 22.7°±0.2°.

Further, the X-ray powder diffraction of the crystal form V has characteristic peaks at the positions where the diffraction angles 2θ are 5.5°±0.2°, 7.1°±0.2°, 8.6°±0.2°, 11.0°±0.2°, 14.3°±0.2°, 15.8°±0.2°, 16.0°±0.2°, 17.9°±0.2°, 19.1°±0.2°, 20.3°±0.2°, 20.5°±0.2°, 21.2°±0.2°, 21.5°±0.2° and 22.7°±0.2°.

Furthermore, the X-ray powder diffraction of the crystal form V has characteristic peaks at the positions where the diffraction angles 2θ are 5.5°±0.2°, 7.1°±0.2°, 8.6°±0.2°, 11.0°± 0.2°, 12.8°±0.2°, 14.3°±0.2°, 15.8°±0.2°, 16.0°±0.2°, 17.9°±0.2°, 19.1°±0.2°, 20.0°±0.2°, 20.3°±0.2°, 20.5°±0.2°, 21.2°±0.2°, 21.5°±0.2°, 22.7°±0.2° and 24.5°±0.2°.

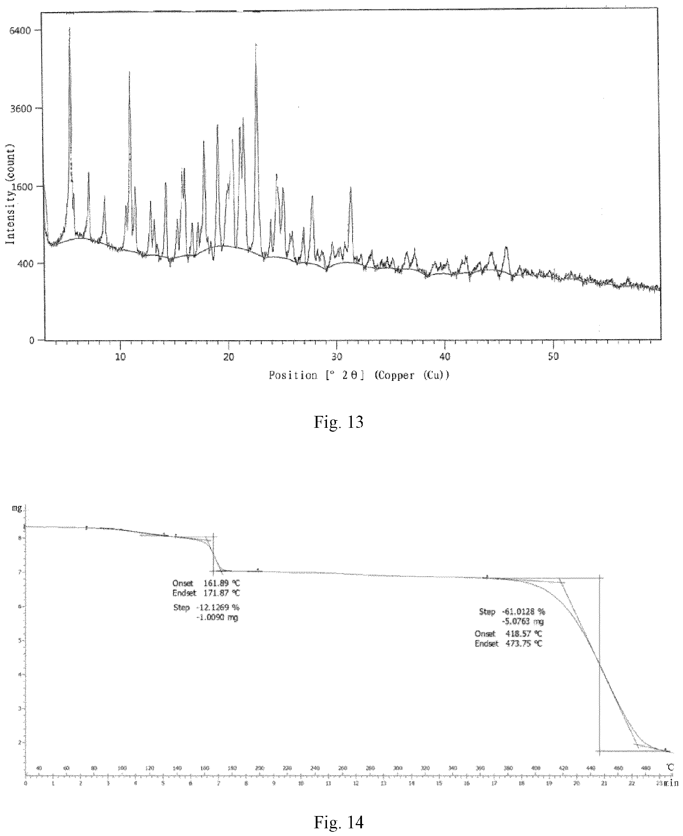

Without limitation, the X-ray powder diffraction pattern of the crystal form V is shown in Fig. 13.

Without limitation, the crystal form V loses 12.13% of its weight at 161.8°C (loss of L-tartaric acid molecules), according to the theoretical calculation, the molar ratio of Apixaban to L-tartaric acid is 1:0.5, and its TGA graph is shown in Fig. 14.

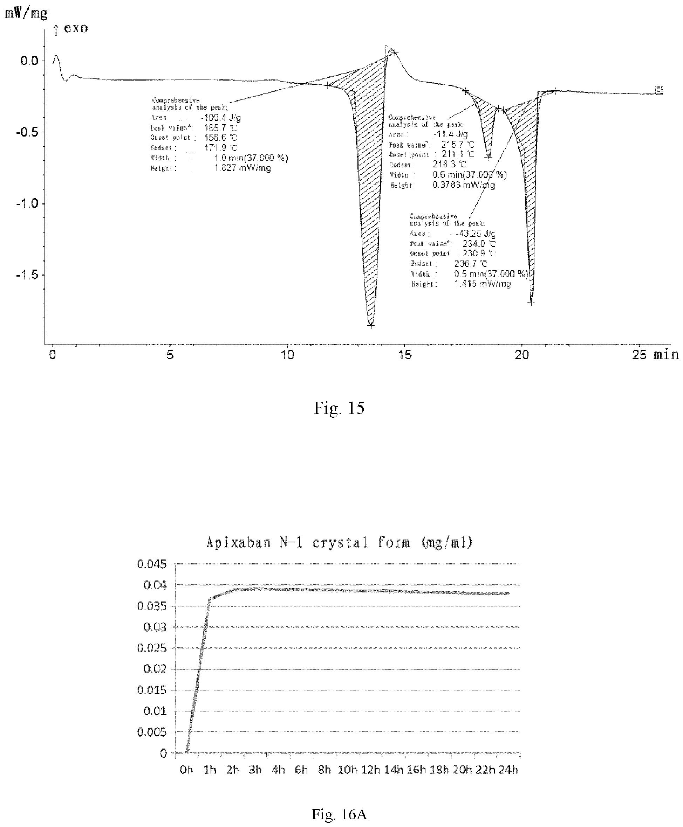

Without limitation, the crystal form V shows endothermic peaks at about 165.7°C, 215.7°C, and 234.0°C, and its DSC graph is shown in Fig. 15.

The crystal form V is a powder with good appearance and fluidity, its dissolution rate is 1.45 times that of Apixaban, and is suitable for being prepared into its oral preparation.

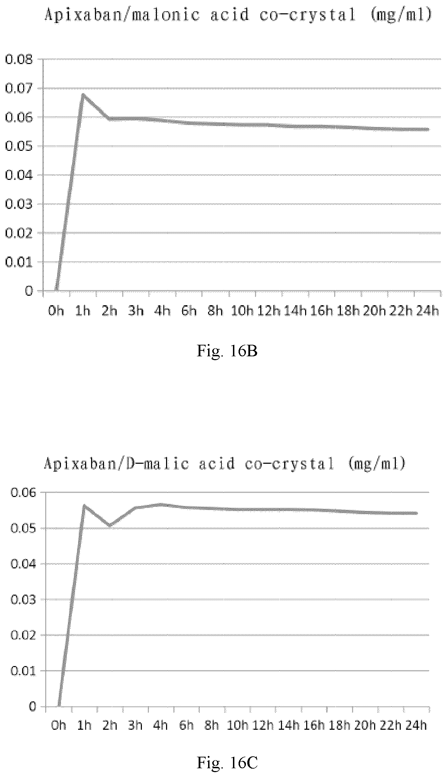

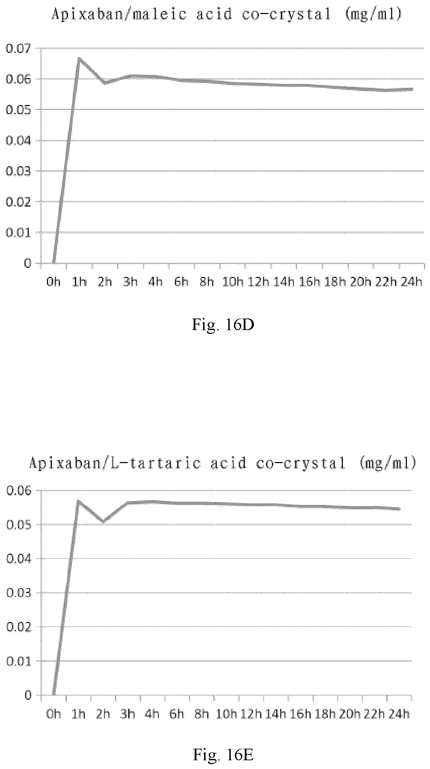

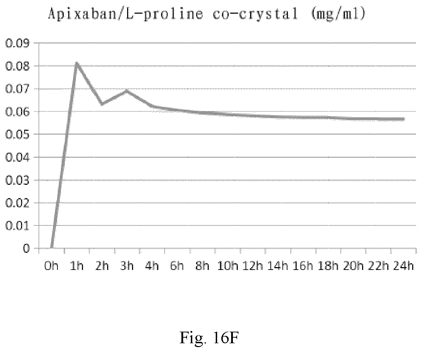

The curves of dissolution rates of the above five Apixaban/carboxylic acid co-crystals are shown in Fig. 16B to Fig. 16F. The results show that the dissolution rates of the five co-crystals are all higher than that of Apixaban, wherein the dissolution rate of Apixaban/proline co-crystal form IV is the highest, which can reach twice that of Apixaban soon, and then gradually decline.

The present invention also provides a method for preparing the above five types of co-crystals, and the method includes:
dissolving the carboxylic acid and Apixaban in a mixed solvent of trifluoroethanol and a poor solvent, stirring for crystallization, filtering, and drying to obtain the Apixaban/carboxylic acid co-crystal.

Preferably, the co-crystal preparation method of the present invention includes:
dissolving the carboxylic acid and Apixaban in a mixed solvent of trifluoroethanol and a poor solvent, stirring at 30-35°C for 18 hours, then cooling to 0-5°C and crystallizing for 2-10 hours, filtering the obtained solid and drying in vacuum at 55°C to obtain the Apixaban/carboxylic acid co-crystal.

In the co-crystal preparation method of the present invention, the molar ratio of Apixaban to the carboxylic acid is 1:0.5-1.1, preferably 1:0.7-1.

In the co-crystal preparation method of the present invention, the weight-volume ratio (g/mL) of Apixaban to trifluoroethanol is 1:5-6.

In the co-crystal preparation method of the present invention, the weight-volume ratio (g/mL) of Apixaban to the poor solvent is 1:2.5-3.0.

In the co-crystal preparation method of the present invention, the poor solvent is selected from methanol, ethanol, n-propanol, isopropanol, n-butanol, isobutanol, or tert-butanol, preferably methanol.

In the co-crystal preparation method of the present invention, the carboxylic acid is selected from malonic acid, D-malic acid, maleic acid, L-proline or L-tartaric acid.

### DESCRIPTION OF THE DRAWINGS

Fig. 1 is an X-ray powder diffraction pattern of Apixaban/malonic acid co-crystal form I;
Fig. 2 is a TGA graph of Apixaban/malonic acid co-crystal form I;
Fig. 3 is a DSC graph of Apixaban/malonic acid co-crystal form I;
Fig. 4 is an X-ray powder diffraction pattern of Apixaban/D-malic acid co-crystal form II;
Fig. 5 is a TGA graph of Apixaban/D-malic acid co-crystal form II;
Fig. 6 is a DSC graph of Apixaban/D-malic acid co-crystal form II;
Fig. 7 is an X-ray powder diffraction pattern of Apixaban/maleic acid co-crystal form III;
Fig. 8 is a TGA graph of Apixaban/maleic acid co-crystal form III;
Fig. 9 is a DSC graph of Apixaban/maleic acid co-crystal form III;
Fig. 10 is an X-ray powder diffraction pattern of Apixaban/L-proline co-crystal form IV;
Fig. 11 is a TGA graph of Apixaban/L-proline co-crystal form IV;
Fig. 12 is a DSC graph of Apixaban/L-proline co-crystal form IV;
Fig. 13 is an X-ray powder diffraction pattern of Apixaban/L-tartaric acid co-crystal form V;
Fig. 14 is a TGA graph of Apixaban/L-tartaric acid co-crystal form V;
Fig. 15 is a DSC graph of Apixaban/L-tartaric acid co-crystal form V;
Fig. 16A to Fig. 16F are respectively the dissolution rate profiles of Apixaban N-1 crystal form and five Apixaban/carboxylic acid co-crystals of the present invention.

### EMBODIMENT

The information of the main raw materials used in the embodiment of the present invention is as follows:

| Name of material | Specification/grade | Manufacturer |
|---|---|---|
| Apixaban | > 99.9% | Zhejiang Tianyu Pharmaceutical Co., Ltd |
| trifluoroethanol | Analytically pure | Aladdin Reagent Company |
| D-malic acid | Analytically pure | Aladdin Reagent Company |
| malonic acid | Analytically pure | Sinopharm Chemical Reagent Company |
| maleic acid | Analytically pure | Sinopharm Chemical Reagent Company |
| L-proline | Analytically pure | Sinopharm Chemical Reagent Company |
| L-tartaric acid | Analytically pure | Sinopharm Chemical Reagent Company |
| methanol | Analytically pure | Aladdin Reagent Company |

The co-crystals obtained in the present invention are characterized by the following instruments:
X-ray powder diffraction pattern was obtained with Panalytical "X'pert Powder" powder X-ray diffractometer, copper palladium, incident wavelength: 1.54 angstroms;
The differential scanning calorimetry graph was obtained with a Mettler-Toledo differential scanning calorimeter, the sample loading amount was 5.00 mg, and the heating rate was 10.00 K/min;
As to the thermogravimetric analysis graph, the TGA data was collected on TA Instruments Q500. In the TGA graph, the abscissa represents the temperature (°C), and the ordinate represents the percentage of weight loss (%).

### Example 1: Preparation of Apixaban/malonic acid co-crystal form I

At room temperature, 10.2 g of malonic acid, 60 g of Apixaban, 350 ml of trifluoroethanol and 170 ml of methanol were added into a reaction flask, stirred for 1 hour and dissolved. The obtained solution was kept at 30-35°C and continue stirring for 18 hours. By controlling the cooling rate of 10-15°C/hour, the crystallization solution was cooled to 0-5°C for crystallization. After 5 hours of crystallization, a large amount of crystal precipitated. The obtained crystal was filtered and washed with a small amount of methanol, and then vacuum dried at 55°C for 24 hours to obtain 53.5g of Apixaban/malonic acid co-crystal. After testing, the obtained co-crystal is Apixaban/malonic acid co-crystal form I. The X-ray powder diffraction data of the sample is shown in Fig. 1 and Table 1; the TGA graph is shown in Fig. 2; and the DSC graph is shown in Fig. 3.

**Table 1**

| 2θ (°) | Relative intensity (%) |
|---|---|
| 5.5 | 64 |
| 5.7 | 100 |
| 5.9 | 60 |
| 7.1 | 12 |
| 11.0 | 20 |
| 11.5 | 37 |
| 11.9 | 21 |
| 13.3 | 14 |
| 13.5 | 13 |
| 15.5 | 22 |
| 15.7 | 34 |
| 16.0 | 35 |
| 17.4 | 35 |
| 19.1 | 23 |
| 19.7 | 19.4 |
| 20.2 | 31 |
| 20.4 | 22 |
| 21.0 | 66 |
| 21.4 | 49 |
| 21.8 | 30 |
| 22.4 | 42 |
| 23.1 | 12 |
| 24.1 | 29 |
| 24.7 | 17 |
| 25.8 | 14 |
| 30.8 | 15 |

### Example 2: Preparation of Apixaban/D-malic acid co-crystal form II

At room temperature, 13.2 g of D-malic acid, 60 g of Apixaban, 350 ml of trifluoroethanol and 170 mL of methanol were added into a reaction flask, stirred for 1 hour and dissolved. The obtained solution was kept at 30-35°C and continue stirring for 18 hours. By controlling the cooling rate of 10-15°C/hour, the crystallization solution was cooled to 0-5°C for crystallization. After 6 hours of crystallization, a large amount of crystal precipitated. The obtained crystal was filtered and washed with a small amount of methanol, and then vacuum dried at 55°C for 24 hours to obtain 48.6 g of Apixaban/D-malic acid co-crystal. After testing, the obtained co-crystal is Apixaban/D-malic acid co-crystal form II. The X-ray powder diffraction data of the sample is shown in Fig. 4 and Table 2; the TGA graph is shown in Fig. 5; and the DSC graph is shown in Fig. 6.

**Table 2**

| 2θ (°) | Relative intensity (%) |
|---|---|
| 5.7 | 95 |
| 8.7 | 13 |
| 11.5 | 100 |
| 13.2 | 37 |
| 15.8 | 29 |
| 16.0 | 23 |
| 16.2 | 12 |
| 16.5 | 18 |
| 17.6 | 34 |
| 18.5 | 23 |
| 20.1 | 63 |
| 20.7 | 11 |
| 21.8 | 88 |
| 23.1 | 35 |
| 24.6 | 11 |
| 25.1 | 25 |
| 25.8 | 16 |
| 28.9 | 10 |

### Example 3: Preparation of Apixaban/maleic acid co-crystal form III

At room temperature, 11.3 g of maleic acid, 60 g of Apixaban, 350 ml of trifluoroethanol and 170 mL of methanol were added into a reaction flask, stirred for 1 hour and dissolved. The obtained solution was kept at 30-35°C and continue stirring for 18 hours. By controlling the cooling rate of 10-15°C/hour, the crystallization solution was cooled to 0-5°C for crystallization. After 5 hours of crystallization, a large amount of crystal precipitated. The obtained crystal was filtered and washed with a small amount of methanol, and then vacuum dried at 55°C for 24 hours to obtain 45.3g of Apixaban/maleic acid co-crystal. After testing, the obtained co-crystal is Apixaban/maleic acid co-crystal form III. The X-ray powder diffraction data of the sample is shown in Fig. 7 and Table 3; the TGA graph is shown in Fig. 8; and the DSC graph is shown in Fig. 9.

**Table 3**

| 2θ (°) | Relative intensity (%) |
|---|---|
| 5.5 | 64 |
| 5.7 | 100 |
| 6.0 | 60 |
| 7.2 | 11 |
| 11.5 | 37 |
| 11.9 | 21 |
| 13.3 | 14 |
| 13.5 | 12 |
| 15.5 | 22 |
| 15.8 | 34 |
| 16.1 | 35 |
| 16.6 | 5 |
| 17.4 | 35 |
| 19.1 | 23 |
| 19.9 | 19 |
| 20.3 | 31 |
| 21.0 | 66 |
| 21.9 | 30 |
| 22.5 | 41 |
| 23.2 | 12 |
| 24.1 | 29 |
| 24.9 | 17 |
| 25.8 | 14 |

### Example 4: Preparation of Apixaban/L-proline co-crystal form IV

At room temperature, 11.3g of L-proline, 60g of Apixaban, 350ml of trifluoroethanol and 170ml of methanol were added into a reaction flask, stirred for 1 hour and dissolved. The obtained solution was kept at 30-35°C and continue stirring for 18 hours. By controlling the cooling rate of 10-15°C/hour, the crystallization solution was cooled to 0-5°C for crystallization. After 6 hours of crystallization, a large amount of crystal precipitated. The obtained crystal was filtered and washed with a small amount of methanol, and then vacuum dried at 55°C for 24 hours to obtain 54.3 g of Apixaban/L-proline co-crystal. After testing, the obtained co-crystal is Apixaban/L-proline co-crystal form IV. The X-ray powder diffraction data of the sample is shown in Fig. 10 and Table 4; the TGA graph is shown in Fig. 11; and the DSC graph is shown in Fig. 12.

**Table 4**

| 2θ (°) | Relative intensity (%) |
|---|---|
| 5.5 | 43 |
| 5.7 | 40 |
| 5.9 | 100 |
| 7.0 | 10 |
| 11.0 | 18 |
| 11.5 | 10 |
| 11.9 | 28 |
| 13.5 | 20 |
| 15.5 | 18 |
| 16.1 | 20 |
| 17.4 | 40 |
| 17.8 | 15 |
| 19.0 | 28 |
| 20.2 | 13 |
| 20.4 | 13 |
| 21.0 | 43 |
| 21.5 | 25 |
| 21.8 | 11 |
| 22.5 | 33 |
| 22.7 | 15 |
| 24.1 | 16 |
| 25.8 | 11 |
| 30.8 | 16 |

### Example 5: Preparation of Apixaban/L-tartaric acid co-crystal form V

At room temperature, 19.6 g of L-tartaric acid, 60 g of Apixaban, 350 ml of trifluoroethanol and 170 ml of methanol were added into a reaction flask, stirred for 1 hour and dissolved. The obtained solution was kept at 30-35°C and continue stirring for 18 hours. By controlling the cooling rate of 10-15°C/hour, the crystallization solution was cooled to 0-5°C for crystallization. After 6 hours of crystallization, a large amount of crystal precipitated. The obtained crystal was filtered and washed with a small amount of methanol, and then vacuum dried at 55°C for 24 hours to obtain Apixaban/L-tartaric acid co-crystal. After testing, the obtained co-crystal is Apixaban/L-tartaric acid co-crystal form V. The X-ray powder diffraction data of the sample is shown in Fig. 13 and Table 5; the TGA graph is shown in Fig. 14; and the DSC graph is shown in Fig. 15.

**Table 5**

| 2θ (°) | Relative intensity (%) |
|---|---|
| 5.5 | 100 |
| 5.7 | 12 |
| 7.1 | 19 |
| 8.6 | 12 |
| 10.6 | 10 |
| 11.0 | 64 |
| 11.4 | 16 |
| 12.8 | 12 |
| 14.3 | 18 |
| 15.8 | 22 |
| 16.0 | 23 |
| 17.9 | 30 |
| 19.1 | 35 |
| 19.9 | 13 |
| 20.0 | 18 |
| 20.3 | 36 |
| 20.5 | 46 |
| 21.2 | 40 |
| 21.5 | 44 |
| 22.7 | 87 |
| 24.5 | 22 |
| 24.7 | 14 |
| 25.1 | 18 |
| 25.2 | 15 |
| 27.8 | 15 |
| 31.4 | 18 |

### Test Example 1: Dissolution rate test of Apixaban/carboxylic acid co-crystal

According to the dissolution rate test guidelines in the current Chinese Pharmacopoeia, a dissolution rate experiment was designed to respectively investigate the dissolution rates of the five Apixaban/carboxylic acid co-crystals prepared by the present invention. The specific scheme is as follows:

Five Apixaban/carboxylic acid co-crystals of the present invention and Apixaban N-1 crystal form (prepared according to the method disclosed in patent CN101065379A) were sampled, and studies of equilibrium solubility in a dissolution medium (0.1mol/L hydrochloric acid) were conducted.

**Table 6 Dissolution equipment and parameters**

| Dissolution tester | Agilent 708-DS+850-DS |
|---|---|
| Method | paddle method |
| Temperature | 37±0.5°C |
| Rotation rate | 100rpm |
| Volume of dissolution medium | 900ml |
| Sample volume | 1.5ml |
| Prime volume | 10ml |
| Purge volume | 10ml |
| Waste drop volume | 0.5ml |
| Sampling time point | 1h, 2h, 3h, 4h, 6h, 8h, 10h, 12h, 14h, 16h, 18h, 20h, 22h, 24h |

**Table 7 Materials and equipment**

| |
|---|
| Active pharmaceutical ingredient of Apixaban |
| Polyvinylidene fluoride filter membrane (PVDF), 0.45 µm |
| Potassium dihydrogen phosphate |
| Sodium hydroxide |
| Phosphoric acid |
| Hydrochloric acid |

### Preparation of the dissolution medium

0.1mol/L hydrochloric acid solution: 9ml of hydrochloric acid was added into 1000ml of water and stirred well.

### Preparation of solution with saturated solubility

After the dissolution conditions were reached, 500 mg of sample powder were weighed and put into a dissolution cup, rotated, and immediately counted the time, after 1, 2, 3, 4, 6, 8, 10, 12, 14, 16, 18, 20, 22 and 24 hours, sampled with a sampling needle equipped with a 10µm polymeric polyethylene filter membrane, and filtered on-line through a 0.45µm polyvinylidene fluoride (PVDF) filter membrane.

The results are shown in Fig. 16A to Fig. 16F. It can be seen from Fig. 16A to Fig. 16F that the dissolution rates of the five co-crystals of the present invention are all higher than that of Apixaban N-1 crystal form, wherein the dissolution rate of Apixaban/L-proline co-crystal form IV is the highest.

The method of the present invention has been described through the preferred examples. It is obvious that relevant persons can make changes or appropriate alterations and combinations to the methods and uses described herein within the content, spirit and scope of the present invention. From a technical perspective, several optimizations in the implementation steps should also be regarded as the protection scope of the present invention.

## Claims

1. A co-crystal formed by Apixaban and a carboxylic acid, wherein the molar ratio of the Apixaban to the carboxylic acid is 1:0.5.

2. The co-crystal according to claim 1, wherein the carboxylic acid is malonic acid, D-malic acid, maleic acid, L-proline or L-tartaric acid.

3. The co-crystal according to claim 1 or 2, wherein the Apixaban and malonic acid form a co-crystal form I represented by Formula 1: 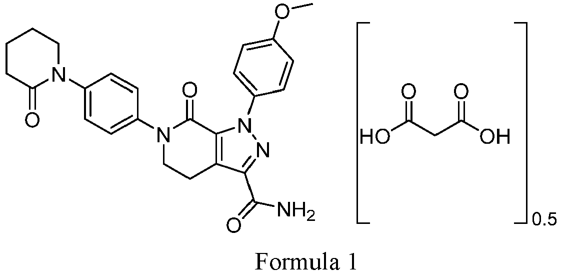 by using Cu-Ka radiation, the X-ray powder diffraction of the crystal form I has characteristic peaks at the positions where the diffraction angles 2θ are 5.5°±0.2°, 5.7°±0.2°, 5.9°±0.2°, 11.0°±0.2°, 11.5°±0.2°, 11.9°±0.2° and 21.0°±0.2°.

4. The co-crystal according to claim 3, wherein the X-ray powder diffraction of the crystal form I has characteristic peaks at the positions where the diffraction angles 2θ are 5.5°±0.2°, 5.7°±0.2°, 5.9°±0.2°, 7.1°±0.2°, 11.0°±0.2°, 11.5°±0.2°, 11.9°±0.2°, 15.5°±0.2°, 15.7°±0.2°, 16.0°±0.2°, 17.4°±0.2°, 21.0°±0.2° and 21.4°±0.2°.

5. The co-crystal according to claim 3, wherein the X-ray powder diffraction of the crystal form I has characteristic peaks at the positions where the diffraction angles 2θ are 5.5°±0.2°, 5.7°±0.2°, 5.9°±0.2°, 7.1°±0.2°, 11.0°±0.2°, 11.5°±0.2°, 11.9°±0.2°, 13.3°±0.2°, 13.5°±0.2°, 15.5°±0.2°, 15.7°±0.2°, 16.0°±0.2°, 17.4°±0.2°, 19.1°±0.2°, 20.2°±0.2°, 21.0°±0.2°, 21.4°±0.2°, 21.8°±0.2°, 22.4°±0.2° and 24.1°±0.2°.

6. The co-crystal according to claim 1 or 2, wherein the Apixaban and D-malic acid form a co-crystal form II represented by Formula 2: 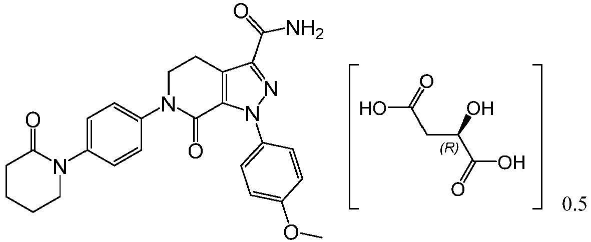 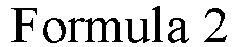 by using Cu-Ka radiation, the X-ray powder diffraction of the crystal form II has characteristic peaks at the positions where the diffraction angles 2θ are 5.7°±0.2°, 8.7°±0.2°, 11.5°±0.2°, 13.2°±0.2°, 17.6°±0.2°, 20.1°±0.2° and 21.8°±0.2°.

7. The co-crystal according to claim 6, wherein the X-ray powder diffraction of the crystal form II has characteristic peaks at the positions where the diffraction angles 2θ are 5.7°±0.2°, 8.7°±0.2°, 11.5°±0.2°, 13.2°±0.2°, 15.8°±0.2°, 16.0°±0.2°, 17.6°±0.2°, 18.5°±0.2°, 20.1°±0.2°, 21.8°±0.2°, 23.1°±0.2° and 25.1°±0.2°.

8. The co-crystal according to claim 6, wherein the X-ray powder diffraction of the crystal form II has characteristic peaks at the positions where the diffraction angles 2θ are 5.7°±0.2°, 8.7°±0.2°, 11.5°±0.2°, 13.2°±0.2°, 15.8°±0.2°, 16.0°±0.2°, 16.2°±0.2°, 16.5°±0.2°, 17.6°±0.2°, 18.5°±0.2°, 20.1°±0.2°, 21.8°±0.2°, 23.1°±0.2°, 25.1°±0.2° and 25.8°±0.2°.

9. The co-crystal according to claim 1 or 2, wherein the Apixaban and maleic acid form a co-crystal form III represented by Formula 3: 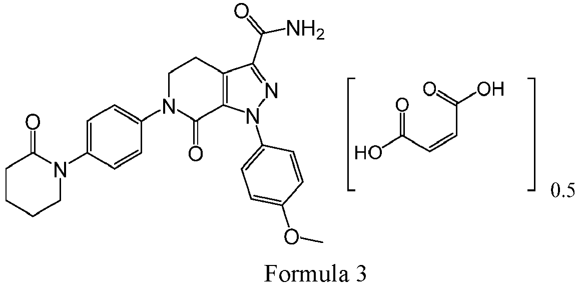 by using Cu-Ka radiation, the X-ray powder diffraction of the crystal form III has characteristic peaks at the positions where the diffraction angles 2θ are 5.5°±0.2°, 5.7°±0.2°, 6.0°±0.2°, 7.2°±0.2°, 11.5°±0.2 °, 15.8°±0.2°, 16.1°±0.2°, 17.4°±0.2° and 21.0°±0.2°.

10. The co-crystal according to claim 9, wherein the X-ray powder diffraction of the crystal form III has characteristic peaks at the positions where the diffraction angles 2θ are 5.5°±0.2°, 5.7°±0.2°, 6.0°±0.2°, 7.2°±0.2°, 11.5°±0.2°, 11.9°±0.2°, 13.3°±0.2°, 13.5°±0.2°, 15.8°±0.2°, 16.1°±0.2°, 17.4°±0.2°, 19.1°±0.2°, 19.9°±0.2°, 20.3°±0.2° and 21.0°±0.2°.

11. The co-crystal according to claim 9, wherein the X-ray powder diffraction of the crystal form III has characteristic peaks at the positions where the diffraction angles 2θ are 5.5°±0.2°, 5.7°±0.2°, 6.0°±0.2°, 7.2°±0.2°, 11.5°±0.2°, 11.9°±0.2°, 13.3°±0.2°, 13.5°±0.2°, 15.8°±0.2°, 16.1°±0.2°, 17.4°±0.2°, 19.1°±0.2°, 19.9°±0.2°, 20.3°±0.2°, 21.0°±0.2°, 21.9°±0.2°, 22.5°±0.2° and 24.1°±0.2°.

12. The co-crystal according to claim 1 or 2, wherein the Apixaban and L-proline form a co-crystal form IV represented by Formula 4: 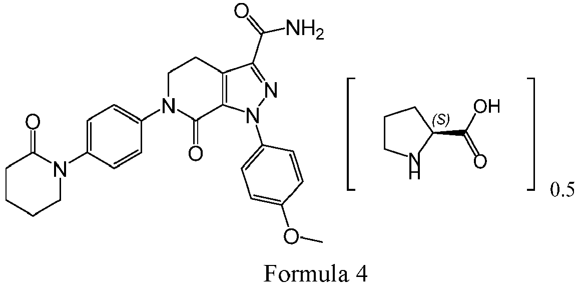 by using Cu-Ka radiation, the X-ray powder diffraction of the crystal form IV has characteristic peaks at the positions where the diffraction angles 2θ are 5.5°±0.2°, 5.7°±0.2°, 5.9°±0.2°, 7.0°±0.2°, 11.9°±0.2°, 17.4°±0.2°, 19.0°±0.2° and 21.0°±0.2°.

13. The co-crystal according to claim 12, wherein the X-ray powder diffraction of the crystal form IV has characteristic peaks at the positions where the diffraction angles 2θ are 5.5°±0.2°, 5.7°±0.2°, 5.9°±0.2°, 7.0°± 0.2°, 11.0°±0.2°, 11.9°±0.2°, 13.5°±0.2°, 15.5°±0.2°, 16.1°±0.2°, 17.4°±0.2°, 17.8°±0.2°, 19.0°±0.2° and 21.0°±0.2°.

14. The co-crystal according to claim 12, wherein the X-ray powder diffraction of the crystal form IV has characteristic peaks at the positions where the diffraction angles 2θ are 5.5°±0.2°, 5.7°±0.2°, 5.9°±0.2°, 7.0°±0.2°, 11.0°±0.2°, 11.9°±0.2°, 13.5°±0.2°, 15.5°±0.2°, 16.1°±0.2°, 17.4°±0.2°, 17.8°±0.2°, 19.0°±0.2°, 21.0°±0.2°, 21.5°±0.2°, 22.5°±0.2° and 24.1°±0.2°.

15. The co-crystal according to claim 1 or 2, wherein the Apixaban and L-tartaric acid form a co-crystal form V represented by Formula 5: 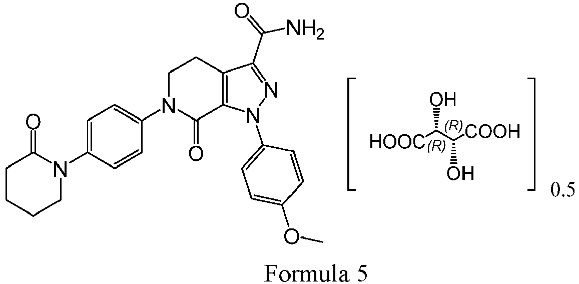 by using Cu-Ka radiation, the X-ray powder diffraction of the crystal form V has characteristic peaks at the positions where the diffraction angles 2θ are 5.5°±0.2°, 7.1°±0.2°, 8.6°±0.2°, 11.0°±0.2°, 17.9°±0.2°, 19.1°±0.2° and 22.7°±0.2°.

16. The co-crystal according to claim 15, wherein the X-ray powder diffraction of the crystal form V has characteristic peaks at the positions where the diffraction angles 2θ are 5.5°±0.2°, 7.1°±0.2°, 8.6°±0.2°, 11.0°± 0.2°, 14.3°±0.2°, 15.8°±0.2°, 16.0°±0.2°, 17.9°±0.2°, 19.1°±0.2°, 20.3°±0.2°, 20.5°±0.2°, 21.2°±0.2°, 21.5°±0.2° and 22.7°±0.2°.

17. The co-crystal according to claim 15, wherein the X-ray powder diffraction of the crystal form V has characteristic peaks at the positions where the diffraction angles 2θ are 5.5°±0.2°, 7.1°±0.2°, 8.6°±0.2°, 11.0°±0.2°, 12.8°±0.2°, 14.3°±0.2°, 15.8°±0.2°, 16.0°±0.2°, 17.9°±0.2°, 19.1°±0.2°, 20.0° ± 0.2°, 20.3°±0.2°, 20.5°±0.2°, 21.2°±0.2°, 21.5°±0.2°, 22.7°±0.2° and 24.5°±0.2°.

18. A method for preparing the co-crystal according to any one of the preceding claims 1-17, including:
dissolving the carboxylic acid and Apixaban in a mixed solvent of trifluoroethanol and a poor solvent, stirring for crystallization, filtering, and drying to obtain the Apixaban/carboxylic acid co-crystal.

19. The method according to claim 18, wherein dissolving the carboxylic acid and Apixaban in a mixed solvent of trifluoroethanol and a poor solvent, stirring at 30-35°C for 18 hours, then cooling to 0-5°C and crystallizing for 2-10 hours, filtering the obtained solid and drying in vacuum at 55°C to obtain the Apixaban/carboxylic acid co-crystal.

20. The method according to claim 18, wherein the molar ratio of Apixaban to the carboxylic acid is 1:0.5-1.1, preferably 1:0.7-1; the weight-volume ratio of Apixaban to trifluoroethanol is 1:5-6; the weight-volume ratio of Apixaban to the poor solvent is 1:2.5-3.0.

21. The method according to claim 18, wherein the poor solvent is selected from methanol, ethanol, n-propanol, isopropanol, n-butanol, isobutanol, or tert-butanol, preferably methanol; the carboxylic acid is selected from malonic acid, D-malic acid, maleic acid, L-proline or L-tartaric acid.
